# EUROPÄISCHE PATENTSCHRIFT

(11) **EP 3 201 604 B1**
(45) Veröffentlichungstag und Bekanntmachung des Hinweises auf die Patenterteilung: **31.10.2018**
(21) Anmeldenummer: 15774542.3
(22) Anmeldetag: 23.09.2015
(51) Int. Cl.: G01N 21/39, G01J 3/433, H01S 5/062, G01N 33/00, G01J 3/42

(54) **VERFAHREN UND GASANALYSATOR ZUR MESSUNG DER KONZENTRATION EINER GASKOMPONENTE IN EINEM MESSGAS**
METHOD AND GAS ANALYSER FOR MEASURING THE CONCENTRATION OF A GAS COMPONENT IN A GAS TO BE MEASURED
PROCÉDÉ ET ANALYSEUR DE GAZ POUR LA MESURE DE LA CONCENTRATION D'UN COMPOSANT DE GAZ DANS UN GAZ DE MESURE

(30) Priorität: 29.09.2014 EP 14186890
(43) Veröffentlichungstag der Anmeldung: 09.08.2017
(73) Patentinhaber: Siemens Aktiengesellschaft, 80333 München (DE)
(72) Erfinder: STEINBACHER, Franz, 76137 Karlsruhe (DE)
(86) Internationale Anmeldenummer: PCT/EP2015/071841
(87) Internationale Veröffentlichungsnummer: WO 2016/050577

(56) Entgegenhaltungen:
- US-B1- 6 351 309
- US-B1- 7 969 576
- R. EICHHOLZ ET AL: "Frequency modulation spectroscopy with a THz quantum-cascade laser", OPTICS EXPRESS, Bd. 21, Nr. 26, 19. Dezember 2013 (2013-12-19), Seite 32199, XP055179888, DOI: 10.1364/OE.21.032199
- SUN H C ET AL: "COMBINED WAVELENGTH AND FREQUENCY MODULATION SPECTROSCOPY: A NOVEL DIAGNOSTIC TOOL FOR MATERIALS PROCESSING", APPLIED OPTICS, OPTICAL SOCIETY OF AMERICA, WASHINGTON, DC; US, Bd. 32, Nr. 6, 20. Februar 1993 (1993-02-20), Seiten 885-893, XP000344962, ISSN: 0003-6935, DOI: 10.1364/AO.32.000885

## Beschreibung

Die Erfindung betrifft ein Verfahren nach dem Oberbegriff des Anspruchs 1 und einen Gasanalysator nach dem Oberbegriff des Anspruchs 7.

Ein derartiges Verfahren bzw. ein derartiger Gasanalysator in Form eines Laserspektrometers sind gleichermaßen aus
D1: US 7 969 576 B1,
D2: R. Eichholz et al: "Frequency modulation spectroscopy with a THz quantum-cascade laser", Optics Express 21(26), 32199 (2013),
D3: US 6 351 309 B1 und
D4: H. C. Sun et al: " Combined wavelength and frequency modulation spectroscopy: a novel diagnostic tool for materials processing", Applied Optics 32(6), 885-893 (1993)
bekannt.

Laserspektrometer werden insbesondere für die optische Gasanalyse in der Prozessmesstechnik eingesetzt. Dabei erzeugt eine Laserdiode Licht im Infrarotbereich, das entlang einer Messstrecke in einer Prozessanlage oder einer Gaszelle durch ein Prozessgas (Messgas) geführt und anschließend detektiert wird. Die Wellenlänge des Lichts wird auf eine spezifische Absorptionslinie der jeweils zu messenden Gaskomponente abgestimmt, wobei die Laserdiode die Absorptionslinie periodisch wellenlängenabhängig abtastet. Dazu wird die Laserdiode innerhalb von aufeinanderfolgenden Abtastintervallen mit einem rampen- oder dreieckförmigen Stromsignal (Injektionsstrom) angesteuert.

Bei der direkten Absorptionsspektroskopie (DAS) wird das von dem Detektor erzeugte Messsignal unmittelbar ausgewertet, wobei die Konzentration der zu messenden Gaskomponente unmittelbar aus der an der Stelle der Absorptionslinie detektierten Minderung der Lichtintensität (Absorption) bestimmt wird. Von Nachteil ist, dass die Detektion in einem extrem niederfrequenten Bereich erfolgt, in dem das Rauschen des Gasanalysators (Laserrauschen, Detektorrauschen) sowie das Rauschen aus der Messstrecke (verursacht durch Turbulenzen, Partikel) sehr hoch sind.

Zur Vermeidung dieses Problems wird der Injektionsstrom für die Laserdiode zusätzlich mit einer vorgegebenen Frequenz und Amplitude sinusförmig moduliert. Bei der Wellenlängenmodulationsspektroskopie (WMS) erfolgt diese Modulation mit einer Frequenz, die viel kleiner als die Halbwertsbreite (FWHM = Full Width at Half Maximum) der Absorptionslinie ist, üblicherweise im kHz-Bereich. Die Modulationsamplitude ist im Vergleich zu dem rampen- oder dreieckförmigen Stromsignal klein, andererseits aber so groß, dass die resultierende spektrale Modulationsamplitude des Laserlichts größer als die Halbwertsbreite (FWHM = Full Width at Half Maximum) der Absorptionslinie ist. Da das Profil der Absorptionslinie nicht linear ist, werden auch Harmonische höherer Ordnung in dem Messsignal erzeugt. Das Messsignal wird üblicherweise bei einer n-ten Oberschwingung, vorzugsweise der zweiten Harmonischen, durch phasensensitive Lock-in Technik demoduliert und für jedes Abtastintervall zu einem Messergebnis ausgewertet. Wegen der geringen Modulationsamplitude ist die Detektion der n-ten Harmonischen direkt proportional zu der n-ten Ableitung des direkten Messsignals. Für den idealen Fall einer lorentzförmigen Absorptionslinie ist z. B. das 2f-Messsignal bei einem Modulationsindex von 2,2 maximal (der Modulationsindex ist das Verhältnis der spektralen Modulationsamplitude zur Halbwertsbreite der abgetasteten Absorptionslinie). Die weitere Auswertung kann z. B. durch Anfitten (Curve-Fitting) des im Idealfall zu erwartenden und mittels eines Näherungsmodells analytisch beschriebenen Verlaufs des demodulierten Messsignals (Sollkurve) an dessen tatsächlichen Verlauf (Istkurve) erfolgen. Da einer der Parameter des Näherungsmodells zu der Konzentration der Gaskomponente proportional ist, erhält man als Ergebnis der Auswertung, und damit als Messergebnis, die Konzentration der zu messenden Gaskomponente.

Bei der Frequenzmodulationsspektroskopie (FMS) wird der Injektionsstrom für die Laserdiode mit einer sehr hohen Frequenz moduliert, die mit der Halbwertsbreite der Absorptionslinie vergleichbar oder größer als diese ist und daher von mehreren 10 MHz bis in den GHz-Bereich reichen kann. Die HF-Modulation erzeugt beiderseits der Emissionsfrequenz der Laserdiode Seitenbänder, die von der Emissionsfrequenz um ganzzahlige Vielfache der Modulationsfrequenz beabstandet sind. Der Modulationsindex ist geringer als bei der WMS und so klein gewählt, dass nur die beiden ersten Seitenbänder des modulierten Laserlichts eine signifikante Amplitude haben. Mit diesen Seitenbändern wird die Absorptionslinie untersucht. Wie auch bei der WMS führt die FMS neben der Modulation der Wellenlänge auch zu einer Modulation der Intensität des Laserlichts, wobei die Wellenlängenmodulation dominiert und die Amplitudenmodulation nur wenig zu dem Messsignal beiträgt. Für die FMS verwendbare Laserdioden (z. B. Bleisalz-Laser oder Quantenkaskadenlaser) müssen daher bei den genannten hohen Frequenzen wellenlängenmodulierbar sein und auch die Detektoren müssen eine sehr große Bandbreite aufweisen. Die Komponenten und der Aufbau eines FM-Spektrometers sind daher sehr teuer und aufwendig. Um Detektoren mit geringerer Bandbreite verwenden zu können, wird bei der Zweiton-FMS die Laserdiode bei zwei dicht beieinander liegenden hohen Frequenzen moduliert, die Detektion erfolgt bei der Schwebungsfrequenz.

DAS, WMS und FMS haben spezifische Vor- und Nachteile. WMS und FMS sind insbesondere bei der Messung geringer Konzentrationen im Vorteil, weil sie Rauschen aus dem Messsignal besser ausfiltern können. Bei höheren Konzentrationen werden aber die für die Auswertung des Messsignals notwendigen Näherungen zunehmend ungenau, wodurch der Messfehler steigt. Die FMS ist zudem sehr teuer und aufwendig. Bei der DAS ist es umgekehrt; aufgrund der höheren Rauschempfindlichkeit ist der Messfehler bei kleinen Konzentrationen höher. Da aber keine Näherungsbeschreibung der Absorptionslinie notwendig ist, wird die Messgenauigkeit mit zunehmender Konzentration besser, weil das Nutzsignal stärker wird. Erst bei sehr hohen Konzentrationen (Sättigung der Absorption) wird das Messverfahren wieder ungenauer.

Das aus der oben genannten US 7 969 576 B1 bekannte Verfahren bzw. der Gasanalysator arbeiten auf der WMS-Basis, wobei neben der n-ten Harmonischen des Messsignals auch seine erste Harmonische, also die Grundfrequenz der Modulation, ausgewertet wird, um das Messergebnis zu normalisieren.

Die Nachweis- und Bestimmungsgrenze für die Messung der Konzentration der Gaskomponente wird durch Rauschen begrenzt, welches dem Messsignal überlagert ist und sich hauptsächlich aus dem Rauschen des Gasanalysators (Laserrauschen, Detektorrauschen) sowie dem Rauschen aus der Messstrecke (verursacht durch Turbulenzen, Partikel) zusammensetzt. Je länger die Messstrecke ist, umso größer sind die Absorption und das erhaltene Messsignal. Sollen geringe Konzentrationen gemessen werden, benötigt man eine ausreichend lange Messstrecke.

Der Erfindung liegt die Aufgabe zugrunde, das Messsignal-Rausch-Verhältnis zu verbessern und so bei gleicher Messstrecke eine deutlich niedrigere Nachweisgrenze zu erreichen.

Gemäß der Erfindung wird die Aufgabe durch das in Anspruch 1 definierte Verfahren sowie den in Anspruch 7 angegebenen Gasanalysator gelöst.

Vorteilhafte Weiterbildungen der Erfindung sind in den Unteransprüchen angegeben.

Entsprechend der Erfindung wird die Laserdiode mit einer Frequenz moduliert, die in Abhängigkeit von den Eigenschaften der Laserdiode so hoch gewählt ist, dass im Unterschied zu WMS und FMS keine Wellenlängenmodulation des erzeugten Lichts stattfindet. Die Wellenlänge des Lichts wird über die innere Temperatur der Laserdiode eingestellt bzw. verändert, wobei diese innere Temperatur wiederum über die Verlustleistung durch den Laserstrom und über die Umgebungstemperatur einstellbar bzw. veränderbar ist. Eine Wellenlängenmodulation kann daher nur mit niedrigen Modulationsfrequenzen, maximal im kHz-Bereich, erfolgen. Bei höheren Frequenzen wird dagegen nur noch die Intensität des Lichts, nicht aber seine Wellenlänge moduliert. So endet bei den derzeit erhältlichen VCSE-Lasern die Wellenlängenmodulation bei einigen MHz (J. Chen et al: "Experimental characterization of the frequency modulation behavior of vertical cavity surface emitting lasers", Applied Physics Letters 91, 141105 (2007)).

Durch die erfindungsgemäße hochfrequente (HF-)Modulation wird das Basisband des auszuwertenden Messsignals aus dem durch das Rauschen des Gasanalysators und der Messstrecke gestörten Frequenzbereich nahe DC in einen hohen Frequenzbereich kopiert, in dem dieses Rauschen nicht mehr vorhanden ist. Das bei der Frequenz der HF-Modulation demodulierte Messsignal enthält demnach dieselben analytischen Informationen (Nutzinformationen) wie das aus der herkömmlichen Ansteuerung der Laserdiode ohne die erfindungsgemäße HF-Modulation resultierende Messsignal, und zwar unabhängig davon, ob lediglich eine rampen- oder dreieckförmige Variation des Stroms der Laserdiode zum Zwecke der wellenlängenabhängigen Abtastung der interessierenden Absorptionslinie und zur Auswertung des Messsignals auf Basis der direkten Absorptionsspektroskopie (DAS) oder zusätzlich eine NF-Modulation des Stromes zum Zwecke einer WMS-Auswertung des Messsignals erfolgt. Das bei der Frequenz der HF-Modulation demodulierte Messsignal kann daher auf dieselbe Weise wie das bisher verwendete Messsignal an dessen Stelle oder ergänzend zu diesem ausgewertet werden.

Bei der wellenlängenabhängigen Abtastung der interessierenden Absorptionslinie der Gaskomponente durch die periodische Variation des Stroms der Laserdiode ändert sich der aus der zusätzlichen HF-Modulation der Laserdiode resultierende Signalanteil des Messsignals prozentual in demselben Maße wie der extrem niederfrequente Signalanteil (nahezu Gleichanteil) des Messsignals, welcher sich aus der Ansteuerung der Laserdiode mit dem rampen- oder dreieckförmigen Stromsignal ergibt. Beide Signalanteile können daher einzeln oder zusammen, z. B. nach Addition, gemäß der DAS ausgewertet werden. Dies gilt in analoger Weise auch für die Auswertung des Messsignals auf WMS-Basis. Da bei der HF-Modulation der Laserdiode keine Wellenlängenmodulation des Lichts stattfindet, die die WMS-Auswertung stören würde, kann die Amplitude der HF-Modulation so hoch gewählt werden, wie es der lineare Aussteuerbereich der Laserdiode zulässt. In jedem Fall ist die Amplitude der erfindungsgemäßen HF-Modulation um ein Vielfaches höher, als bei der niederfrequenten WMS-Modulation, wo der Modulationsindex in Abhängigkeit von der Breite der abzutastenden Absorptionslinie zu wählen ist, und der FMS-Modulation, wo der Modulationsindex noch geringer als bei der WMS ist.

Die hinsichtlich der Auswertung des Messsignals unterschiedlichen Messungen auf Basis von DAS und WMS können gleichzeitig in jedem Abtastintervall oder abwechselnd in aufeinanderfolgenden Abtastintervallen erfolgen und ihre Ergebnisse z. B. durch Mittelwertbildung zu dem Messergebnis verknüpft werden.

Die HF-Modulation kann mit nur einer, in vorteilhafter Weise aber auch mit mehreren oder vielen Frequenzen erfolgen, wobei die bei diesen Frequenzen demodulierten Messsignale entweder einzeln auf der Basis von WMS und/oder DAS ausgewertet und danach zu dem Messergebnis beispielsweise durch Datenfusion (Data Fusion, Multi-Sensor Data Fusion) zusammengeführt, im einfachsten Fall addiert, werden oder zuerst zusammengeführt und dann ausgewertet werden. Durch die Zusammenführung der bei mehreren oder vielen Frequenzen demodulierten Messsignale bzw. der Ergebnisse ihrer Einzelauswertungen erhält man entsprechend viel auswertbare Signalenergie. Der Abstand zwischen den für die HF-Modulation verwendeten Frequenzen muss dabei so groß sein, dass sich die auszuwertenden Frequenzbänder nicht überlappen und darüber hinaus durch Bandpassfilterung sauber erfasst werden können. Da im Unterschied zu den enthaltenen Nutzinformationen das Rauschen in den verschiedenen Frequenzbändern nicht korreliert, wird der Signal-Rauschabstand verbessert. Die Amplituden der sich überlagernden HF-Modulationen werden so gewählt, dass die Laserdiode innerhalb ihres linearen Aussteuerbereichs soweit wie möglich ausgesteuert wird.

Die Messung auf Basis von WMS kann in herkömmlicher Weise erfolgen, indem der Strom der Laserdiode und damit die Wellenlänge des erzeugten Lichts mit einer zusätzlichen Frequenz moduliert wird und das Messsignal im Basisband und das demodulierte Messsignal aus dem höheren Frequenzbereich bei einer Harmonischen, insbesondere der zweiten Harmonischen, der zusätzlichen Frequenz ausgewertet werden.

Die WMS kann aber auch modifiziert und erweitert werden, so wie dies z. B. Gegenstand der älteren und noch nicht veröffentlichten deutschen Patentanmeldung mit dem Aktenzeichen DE102014215848.6 ist. Dort erfolgt die niederfrequente WMS-Modulation mit mehreren zusätzlichen Frequenzen, die um den doppelten Betrag der niedrigsten zusätzlichen Frequenz voneinander beabstandet sind, also beispielsweise f_{NF}, 3f_{NF}, 5f_{NF} und 7f_{NF}. Die WMS-Auswertung des Messsignals bzw. des HF-demodulierten Messsignals erfolgt an den Stellen der zweiten Harmonischen dieser Frequenzen, also 2f_{NF}, 6f_{NF}, 10f_{NF} und 14f_{NF}, und mindestens einer ihrer Summen- und Differenzfrequenzen, also 2f_{NF}, 4f_{NF}, 6f_{NF}, 8f_{NF}, 6f_{NF}, ... 14f_{NF}. Wegen der nichtlinearen Form der Absorptionslinie enthält das Messsignal nicht nur die Vielfachen (Harmonischen) der bei der Modulation verwendeten Frequenzen, sondern auch die Summen- und Differenzen dieser Frequenzen. Da die verwendeten WMS-Modulationsfrequenzen um den doppelten Betrag 2f_{NF} der kleinsten Modulationsfrequenz f_{NF} auseinander liegen, fallen ihre Summen- und Differenzfrequenzen entweder mit den zweiten Harmonischen zusammen oder liegen genau in der Mitte zwischen diesen. Daher liegen auch die Frequenzanteile des Messsignals jeweils um den doppelten Betrag 2f_{NF} der kleinsten Modulationsfrequenz f_{NF} auseinander. Die entsprechenden Messsignalanteile weisen jeweils die gleichen Verläufe auf, so dass sie sich in konstruktiver Weise überlagern und es gelingt, entsprechend viel auswertbare Signalenergie aus der Absorption zu erhalten. Da das Rauschen bei den unterschiedlichen Frequenzen im Unterschied zu den sich addierenden Signalanteilen nicht korreliert ist, entsteht bei der Auswertung zu dem Messergebnis ein sehr hoher Signal-Rauschabstand.

Zusätzlich oder ergänzend kann das Messsignal bzw. demodulierte Messsignal bei den Modulationsfrequenzen f_{NF}, 3f_{NF}, 5f_{NF} und 7f_{NF} ausgewertet werden.

Zur weiteren Erläuterung der Erfindung wird im Folgenden auf die Figuren der Zeichnung Bezug genommen; im Einzelnen zeigen:
- Figur 1: ein erstes Ausführungsbeispiel des erfindungsgemäßen Gasanalysators,
- Figur 2: ein Beispiel einer HF-Demodulation des Messsignals, und
- Figur 3: ein weiteres Ausführungsbeispiel des erfindungsgemäßen Gasanalysators.

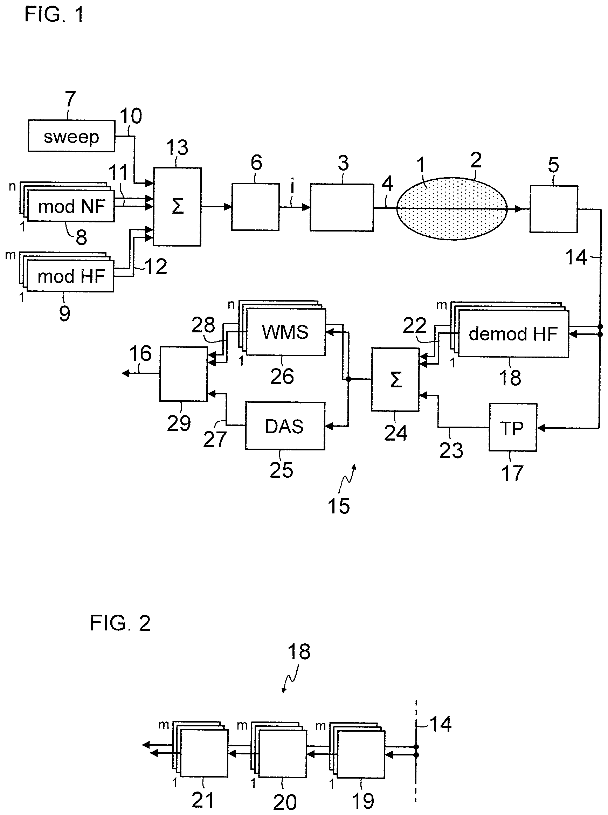

Figur 1 zeigt, in Form eines stark vereinfachten Blockschaltbildes, einen Gasanalysator zur Messung der Konzentration mindestens einer interessierenden Gaskomponente eines Messgases 1, das in einem Messvolumen 2, beispielsweise einer Gaszelle oder einer Prozessgasleitung, enthalten ist. Der Gasanalysator enthält eine Laserdiode 3, deren Licht 4 nach Durchstrahlen des Messgases 1 auf einen Detektor 5 fällt. Eine steuerbare Stromquelle 6 speist die Laserdiode 3 mit einem Injektionsstrom i, wobei die Intensität und die Wellenlänge des erzeugten Lichts 4 von dem Strom i und der Betriebstemperatur der Laserdiode 3 abhängen. Zur Variation und Modulation des Stromes i erzeugen ein Signalgenerator 7, eine Niederfrequenz-(NF-)Modulationseinrichtung 8 und eine Hochfrequenz-(HF-)Modulationseinrichtung 9 unterschiedliche Signale 10, 11, 12, die der Stromquelle 6 über einen Summierer 13 zugeführt werden.

Mit dem Signal 10 des Signalgenerators 7 wird der Strom i periodisch entsprechend einer vorgegebenen, vorzugsweise rampen- oder dreieckförmigen Funktion variiert, um mit der mehr oder weniger linear folgenden Wellenlänge des erzeugten Lichts 4 eine ausgewählte Absorptionslinie der interessierenden Gaskomponente abzutasten. Das Signal 10 kann zusätzlich Bursts enthalten, die in regelmäßigen Abständen, z. B. nach jeder Abtastperiode, aufeinander folgen und später eine Normalisierung der Messung ermöglichen.

Die Niederfrequenz-(NF-)Modulationseinrichtung 8 ist vorhanden, wenn die Konzentration der interessierenden Gaskomponente auf Basis der Wellenlängenmodulationsspektroskopie (WMS) erfolgen soll. In diesem Fall wird der Strom i und damit die Wellenlänge des erzeugten Lichts 4 mit einer Frequenz im kHz-Bereich und kleiner Amplitude sinusförmig moduliert. Wie angedeutet ist, kann die NF-Modulation auf n verschiedene Frequenzen erweitert werden.

Die Hochfrequenz-(HF-)Modulationseinrichtung 8 dient dazu, den Strom i mit einer sehr hohen Frequenz im MHz-Bereich, z. B: 50 MHz, und hoher Amplitude zu modulieren. Die Hochfrequenz ist dabei in Abhängigkeit von den Eigenschaften der verwendeten Laserdiode 3 so gewählt, dass nur die Intensität des erzeugten Lichts 4 moduliert wird und keine Wellenlängenmodulation stattfindet. Die Amplitude der Modulation ist im Rahmen des linearen Aussteuerbereichs der Laserdiode 3 maximal; sie kann beispielsweise im Größenbereich des halben Mittelwerts der Stromrampe (Signal 10) liegen. Wie die NF-Modulation kann auch die HF-Modulation auf mehrere oder viele, hier m, verschiedene Frequenzen erweitert werden. Der Abstand zwischen den Frequenzen muss dabei so groß sein, dass sich die auf WMS-Basis auszuwertenden Frequenzbänder nicht überlappen und nach Detektion durch Bandpassfilterung sauber erfasst werden können.

Der Detektor 5 erzeugt in Abhängigkeit von der detektierten Lichtintensität ein Messsignal 14, das anhand der aus den Bursts des Signals 10 resultierenden Signalanteile automatisch verstärkt und normalisiert werden kann. Im Weiteren wird das Messsignal 14 in einer Auswerteeinrichtung 15 zu einem die Konzentration der interessierenden Gaskomponente in dem Messgas 1 angebenden Messergebnis 16 verarbeitet. Aufgrund der HF-Modulation der Wellenlänge des Lichts 4 wird das Basisband des Messsignals 14 mit den darin enthaltenen Nutzinformationen in höhere Frequenzbereiche kopiert. Die in dem Basisband enthaltenen Nutzinformationen werden durch Filtern des Messsignals 14 in einem Tiefpassfilter 17 mit einer unter der niedrigsten der verwendeten HF-Modulationsfrequenz liegenden Grenzfrequenz erhalten. Um die Nutzinformationen aus den höheren Frequenzbändern zu erhalten, wird das Messsignal 14 parallel zur Tiefpassfilterung in einer HF-Demodulationseinrichtung 18 bei jeder der m (m ≥ 1) verwendeten Frequenzen der HF-Modulation demoduliert.

Wie Figur 2 zeigt, erfolgt die Demodulation in m parallelen Kanälen, die jeweils ein Bandpassfilter 19 und einen Lock-in Verstärker 20 mit nachgeordnetem Tiefpassfilter 21 aufweisen. Dabei wird das bandpassgefilterte Messsignal 14 durch Multiplikation mit einem Referenzsignal bei der jeweiligen HF-Demodulationsfrequenz phasensensitiv demoduliert, und durch die anschließende Tiefpassfilterung die Inphasenkomponente, also der Nutzsignalanteil 22 des demodulierten Messsignals 14 extrahiert. Die Bandbreite des Bandpassfilters 19 und die Grenzfrequenz des Tiefpassfilters 21 sind so groß, dass sie die auf DAS- und/oder WMS-Basis auszuwertenden Frequenzbänder des Messsignals 14 durchlassen.

Zurück zu Figur 1 werden die durch die Tiefpassfilterung und Demodulation erhaltenen Nutzinformationen 23, 22 in einem Summierer 24 addiert und anschließend in einer Recheneinrichtung 25 auf DAS-Basis und/oder in einer weiteren Recheneinrichtung 26 auf WMS-Basis ausgewertet. Die Ergebnisse 27, 28 der DAS- und WMS-Auswertungen werden in einer dritten Recheneinrichtung 29 addiert oder unter Zuhilfenahme der statistischen Signalverarbeitung zusammengeführt, um schließlich als Messergebnis 16 die Konzentration der zu messenden Gaskomponente zu erhalten.

Die WMS-Auswertung erfolgt z. B. an den Stellen der zweiten Harmonischen der n (n ≥ 1) verwendeten Frequenzen der niederfrequenten WMS-Modulation. In diesem Fall handelt es sich um n Einzelauswertungen, die z. B. n Kurvenverläufe ergeben. Darüber hinaus kann die WMS-Auswertung auch an den Stellen der Grundfrequenzen oder weiterer höherer Harmonischer erfolgen, was zu zusätzlichen Sätzen von jeweils n Einzelauswertungen führt. Die n Kurvenverläufe innerhalb jedes einzelnen Satzes korrelieren und können zu einem Zwischenergebnis ausgewertet werden. Die Zwischenergebnisse aus den unterschiedlichen Sätzen werden dann ihrerseits zusammengeführt, im einfachsten Fall addiert.

Die WMS-Auswertung kann erweitert werden, indem die niederfrequente WMS-Modulation bei Frequenzen erfolgt, die um den doppelten Betrag der niedrigsten zusätzlichen Frequenz voneinander beabstandet sind, also für n = 4 die Frequenzen: f_{NF}, 3f_{NF}, 5f_{NF} und 7f_{NF}. Die WMS-Auswertung kann dann sowohl an den Stellen der zweiten Harmonischen dieser Frequenzen, also 2f_{NF}, 6f_{NF}, 10f_{NF} und 14f_{NF}, als auch an den Stellen ihrer Summen- und Differenzfrequenzen, also 2f_{NF}, 4f_{NF}, 6f_{NF}, 8f_{NF}, 6f_{NF}, ... 14f_{NF}, erfolgen. In diesem Fall handelt es sich um 2(2n-1) = 14 Einzelauswertungen, die 14 Kurvenverläufe ergeben. Wie oben bereits erwähnt, enthält das Messsignal 14 wegen der nichtlinearen Form der abgetasteten Absorptionslinie nicht nur die zweiten Harmonischen der bei der Modulation verwendeten Frequenzen, sondern auch die Summen- und Differenzen dieser Frequenzen, die entweder mit den zweiten Harmonischen zusammenfallen oder genau in der Mitte zwischen diesen liegen. Die Messsignalanteile an den Stellen der Frequenzen 2f_{NF}, 4f_{NF}, 6f_{NF}, 8f_{NF}, 6f_{NF}, ... 14f_{NF} weisen jeweils die gleichen Verläufe auf, so dass sie sich konstruktiv überlagern und so die auswertbare Signalenergie aus der Absorption erhöhen.

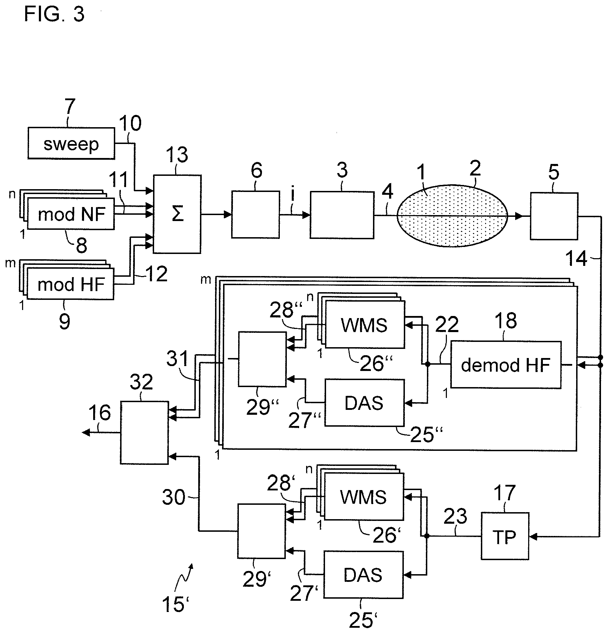

Figur 3 zeigt ein weiteres Ausführungsbeispiel des erfindungsgemäßen Gasanalysators mit einer im Vergleich zu Figur 1 modifizierten Auswerteeinrichtung 15'. Die durch Filtern des Messsignals 14 mittels des Tiefpasses 17 sowie durch Demodulation an den Stellen der m verschiedenen Frequenzen der HF-Modulation enthaltenen Nutzinformationen 23, 22 werden nicht, wie im Falle des Ausführungsbeispiels nach Figur 1, addiert und anschließend ausgewertet, sondern einzeln für den Tiefpasskanal und jeden der m HF-Demodulationskanäle in Recheneinrichtungen 25', 25" auf DAS-Basis und weiteren Recheneinrichtungen 26', 26'' auf WMS-Basis ausgewertet. Zum Schluss werden die Ergebnisse 30, 31 aus den unterschiedlichen in einer Recheneinheit 32, beispielsweise einem Addierer zu dem Messergebnis 16 zusammengeführt.

## Patentansprüche

1. Verfahren zur Messung der Konzentration einer Gaskomponente in einem Messgas (1) mittels eines Gasanalysators, wobei
- eine wellenlängenabstimmbare Laserdiode (3) mit einem Strom (i) angesteuert und das von der Laserdiode (3) erzeugte Licht (4) durch das Messgas (1) auf einen Detektor (5) geführt wird,
- zur wellenlängenabhängigen Abtastung einer interessierenden Absorptionslinie der Gaskomponente der Strom (i) in periodisch aufeinanderfolgenden Abtastintervallen variiert wird,
- der Strom (i) zusätzlich mit einer vorgegebenen Frequenz und Amplitude sinusförmig moduliert wird und
- ein von dem Detektor (5) erzeugtes Messsignal (14) bei der Frequenz der Modulation demoduliert und das dabei erhaltene demodulierte Messsignal (22) zur Erzeugung eines Messergebnisses (16) ausgewertet wird,
**dadurch gekennzeichnet,**
- **dass** die Frequenz der Modulation in Abhängigkeit von den Eigenschaften der Laserdiode (3) so hoch gewählt ist, dass keine Wellenlängenmodulation stattfindet und
- **dass** die Amplitude der Modulation innerhalb und unter Ausnutzung des linearen Aussteuerbereichs der Laserdiode (3) mit maximaler Höhe gewählt ist.

2. Verfahren nach Anspruch 1, **dadurch gekennzeichnet,**
- **dass** der Strom (i) der Laserdiode (3) mit mindestens einer weiteren Frequenz moduliert wird, die ebenfalls so hoch gewählt ist, dass keine Wellenlängenmodulation stattfindet,
- **dass** die Amplituden der Modulationen innerhalb und unter Ausnutzung des linearen Aussteuerbereichs der Laserdiode (3) mit maximaler Höhe gewählt sind,
- **dass** das von dem Detektor (5) erzeugte Messsignal (14) zusätzlich bei der Frequenz der mindestens einen weiteren Modulation demoduliert wird und
- **dass** die erhaltenen demodulierten Messsignale (22) entweder einzeln ausgewertet und anschließend zu dem Messergebnis (16) zusammengeführt oder zuerst zusammengeführt und anschließend zu dem Messergebnis (16) ausgewertet werden.

3. Verfahren nach Anspruch 1 oder 2, basierend auf der direkten Absorptionsspektroskopie.

4. Verfahren nach einem der vorangehenden Ansprüche basierend auf der Wellenlängenmodulationsspektroskopie wobei der Strom (i) der Laserdiode (3) zusätzlich mit geringer Amplitude und mindestens einer zusätzlichen Frequenz moduliert wird, die so niedrig gewählt ist, dass eine Wellenlängenmodulation stattfindet.

5. Verfahren nach Anspruch 3 und 4, **dadurch gekennzeichnet, dass** Messungen auf Basis der direkten Absorptionsspektroskopie und der Wellenlängenmodulationsspektroskopie gleichzeitig in jedem Abtastintervall oder abwechselnd in aufeinanderfolgenden Abtastintervallen erfolgen und ihre Ergebnisse durch Mittelwertbildung zu dem Messergebnis verknüpft werden.

6. Verfahren nach Anspruch 4 oder 5, **dadurch gekennzeichnet, dass** bei Modulation mit mehreren zusätzlichen Frequenzen diese um den doppelten Betrag der niedrigsten zusätzlichen Frequenz voneinander beabstandet sind und dass die auf der Wellenlängenmodulationsspektroskopie basierende Auswertung des demodulierten Messsignals an den Stellen der zweiten Harmonischen der zusätzlichen Frequenzen und mindestens einer der Summen- und Differenzfrequenzen der zusätzlichen Frequenzen erfolgt.

7. Gasanalysator zur Messung der Konzentration einer Gaskomponente in einem Messgas (1) mit
- einer wellenlängenabstimmbaren Laserdiode (3),
- einer die Laserdiode (3) mit einem Strom (i) speisenden Stromquelle (6),
- einem Signalgenerator (7), der die Stromquelle (6) steuert um den Strom (i) zur wellenlängenabhängigen Abtastung einer interessierenden Absorptionslinie der Gaskomponente in periodisch aufeinanderfolgenden Abtastintervallen zu variieren,
- einer Modulationseinrichtung (9), die die Stromquelle (6) steuert um den Strom (i) mit einer vorgegebenen Frequenz und Amplitude sinusförmig zu modulieren,
- Mitteln, die das modulierte Licht (4) durch das Messgas (1) auf einen Detektor (5) führen, und
- einer Auswerteeinrichtung (15, 15'), die ein von dem Detektor (5) erzeugtes Messsignal (14) bei der Frequenz der Modulation demoduliert und das dabei erhaltene demodulierte Messsignal (22) zur Erzeugung eines Messergebnisses (16) auswertet,
**dadurch gekennzeichnet,**
- **dass** die Modulationseinrichtung (9) dazu ausgebildet ist, die Modulation der Frequenz in Abhängigkeit von den Eigenschaften der Laserdiode mit einer so hohen Frequenz durchzuführen, dass keine Wellenlängenmodulation stattfindet, und
- **dass** die Modulationseinrichtung (9) ferner dazu ausgebildet ist, die Modulation der Amplitude innerhalb und unter Ausnutzung des linearen Aussteuerbereichs der Laserdiode mit maximaler Höhe durchzuführen.

8. Gasanalysator nach Anspruch 7, **dadurch gekennzeichnet,**
- **dass** die Modulationseinrichtung (9) ferner dazu ausgebildet ist, den Strom (i) der Laserdiode (3) mit mindestens einer weiteren Frequenz zu modulieren, die ebenfalls so hoch gewählt ist, dass keine Wellenlängenmodulation stattfindet,
- **dass** die Amplituden der Modulationen innerhalb und unter Ausnutzung des linearen Aussteuerbereichs der Laserdiode (3) mit maximaler Höhe gewählt sind,
- **dass** die Auswerteeinrichtung (15, 15') dazu ausgebildet ist, das von dem Detektor (5) erzeugte Messsignal (14) zusätzlich bei der Frequenz der mindestens einen weiteren Modulation zu demodulieren und die erhaltenen demodulierten Messsignale (22) entweder einzeln auszuwerten und anschließend zu dem Messergebnis (16) zusammenzuführen oder zuerst zusammenzuführen und anschließend zu dem Messergebnis (16) auszuwerten.

9. Gasanalysator nach Anspruch 7 oder 8, **dadurch gekennzeichnet, dass** die Auswerteeinrichtung (15, 15') dazu ausgebildet ist, das demodulierte Messsignal (22) auf der Grundlage der direkten Absorptionsspektroskopie auszuwerten.

10. Gasanalysator nach Anspruch 7, 8 oder 9, **dadurch gekennzeichnet,**
- **dass** eine Niederfrequenz-(NF-)Modulationseinrichtung (8) vorhanden ist, die die Stromquelle (6) steuert um den Strom (i) zusätzlich mit geringer Amplitude und mindestens einer zusätzlichen Frequenz zu modulieren, die so niedrig gewählt ist, dass eine Wellenlängenmodulation stattfindet, und
- **dass** die Auswerteeinrichtung (15, 15') dazu ausgebildet ist, das demodulierte Messsignal (14) auf der Grundlage der Wellenlängenmodulationsspektroskopie auszuwerten.

## Claims

1. Method for measuring the concentration of a gas component in a sample gas (1) by means of a gas analyser, wherein
- a wavelength-tunable laser diode (3) is actuated by a current (i) and the light (4) generated by the laser diode (3) is guided through the sample gas (1) to a detector (5),
- for the wavelength-dependent sampling of an absorption line of interest of the gas component, the current (i) is varied in periodically successive sampling intervals,
- the current (i) is additionally modulated sinusoidally with a predefined frequency and amplitude and
- a measuring signal (14) generated by the detector (5) is demodulated at the frequency of modulation and the demodulated measuring signal (22) obtained thereby is evaluated to form a measurement result (16),
**characterised in that**
- the modulation frequency is selected as a function of the properties of the laser diode (3) at a level high enough to ensure that no wavelength modulation takes place and
- the modulation amplitude is selected at the maximum level within and utilising the linear control range of the laser diode (3).

2. Method according to claim 1, **characterised in that**
- the current (i) of the laser diode (3) is modulated with at least one further frequency, which is also selected high enough to ensure that no wavelength modulation takes place,
- the modulation amplitudes within and utilising the linear control range of the laser diode (3) are selected at the maximum level,
- the measuring signal (14) generated by the detector (5) is additionally demodulated at the frequency of the at least one further modulation and
- the demodulated measuring signals (22) obtained are either evaluated individually and then combined to form the measurement result (16) or first combined and then evaluated to form the measurement result (16).

3. Method according to claim 1 or 2 based on direct absorption spectroscopy.

4. Method according to one of the preceding claims based on wavelength modulation spectroscopy wherein the current (i) of laser diode (3) is additionally modulated with a low amplitude and at least one additional frequency which is selected low enough to ensure that wavelength modulation takes place.

5. Method according to claim 3 and 4, **characterised in that** measurements based on direct absorption spectroscopy and wavelength modulation spectroscopy are performed simultaneously in each sampling interval or alternately in successive sampling intervals and the results thereof linked by averaging to form the measurement result.

6. Method according to claim 4 or 5, **characterised in that**, in the case of modulation with a plurality of additional frequencies, said frequencies are spaced apart by twice the magnitude of the lowest additional frequency and that the evaluation of the demodulated measuring signal based on wavelength modulation spectroscopy is performed at the second harmonics of the additional frequencies and at least one of the sum and difference frequencies of the additional frequencies.

7. Gas analyser for measuring the concentration of a gas component in a sample gas (1) with
- a wavelength-tunable laser diode (3),
- a current source (6) that feeds the laser diode (3) with a current (i),
- a signal generator (7) that controls the current source (6) in order to vary the current (i) for the wavelength-dependent sampling of an absorption line of interest of the gas component within periodically successive sampling intervals,
- a modulation apparatus (9), which feeds the current source (6) in order to modulate the current (i) sinusoidally with a predefined frequency and amplitude,
- means to guide the modulated light (4) through the sample gas (1) to a detector (5) and
- an evaluation apparatus (15, 15'), which demodulates a measuring signal (14) generated by the detector (5) at the modulation frequency and evaluates the demodulated measuring signal (22) obtained thereby for the generation of a measurement result (16),
**characterised in that**
- the modulation apparatus (9) is embodied to carry out the modulation of the frequency as a function of the properties of the laser diode with a frequency high enough to ensure that no wavelength modulation takes place and
- the modulation apparatus (9) is further embodied to carry out the modulation of the amplitude within and while making use of the linear control range of the laser diode at the maximum level.

8. Gas analyser according to claim 7, **characterised in that**
- the modulation apparatus (9) is further embodied to modulate the current (i) of the laser diode (3) with at least one further frequency, which is also selected high enough to ensure that no wavelength modulation takes place,
- the modulation amplitudes are selected at the maximum level within and utilising the linear control range of the laser diode (3),
- the evaluation apparatus (15, 15') is embodied to demodulate the measuring signal (14) generated by the detector (5) additionally at the frequency of the at least one further modulation and either evaluate the demodulated measuring signals (22) obtained individually and then combine them to form the measurement result (16) or first combine them and then evaluate them to form the measurement result (16).

9. Gas analyser according to claim 7 or 8, **characterised in that** the evaluation apparatus (15, 15') is embodied to evaluate the demodulated measuring signal (22) on the basis of direct absorption spectroscopy.

10. Gas analyser according to claim 7, 8 or 9, **characterised in that**
- a low-frequency (LF) modulation apparatus (8) is provided which controls the current source (6) in order to modulate the current (i) additionally with a low amplitude and at least one additional frequency which is selected low enough to ensure that wavelength modulation takes place and
- the evaluation apparatus (15, 15') is embodied to evaluate the demodulated measuring signal (14) on the basis of wavelength modulation spectroscopy.

## Revendications

1. Procédé de mesure de la concentration d'un constituant gazeux d'un gaz (1) à mesurer au moyen d'un analyseur de gaz, dans lequel
- on excite, par un courant (i), une diode (3) laser accordable en longueur d'onde et on fait passer la lumière (4) produite par la diode (3) laser dans le gaz (1) à mesurer jusqu'à un détecteur (5),
- pour l'échantillonnage en fonction des longueurs d'onde d'une raie d'absorption intéressante du constituant gazeux, on fait varier le courant (i) à des intervalles d'échantillonnage se succédant périodiquement,
- on module sinusoïdalement le courant (i) supplémentairement à une fréquence et une amplitude données à l'avance et
- on démodule, à la fréquence de modulation, le signal (14) de mesure produit par le détecteur (5) et on exploite le signal (22) de mesure démodulé ainsi obtenu pour produire un résultat (16) de mesure,
**caractérisé**
- **en ce que** l'on choisit la fréquence de la modulation en fonction des propriétés de la diode (3) laser si haute qu'une modulation en longueur d'onde n'a pas lieu et
- **en ce que** l'on choisit l'amplitude de la modulation à un niveau maximum dans et en utilisant la plage de commande linéaire de la diode (3) laser.

2. Procédé suivant la revendication 1, **caractérisé**
- **en ce que** l'on module le courant (i) de la diode (3) laser à au moins une autre fréquence différente, qui est choisie également si haute qu'il ne se produit pas de modulation en longueur d'onde,
- **en ce que** l'on choisit les amplitudes des modulations à un niveau maximum dans et en utilisant la plage de commande linéaire e la diode (3) laser,
- **en ce que** l'on démodule le signal (14) de mesure produit par le détecteur (5), en outre, à la fréquence de la au moins une autre modulation et
- **en ce que** l'on exploite les signaux (22) de mesure démodulés obtenus, soit individuellement, et on les rassemble ensuite en le résultat (16) de mesure ou on les rassemble d'abord et on les exploite ensuite en le résultat (16) de mesure.

3. Procédé suivant la revendication 1 ou 2, reposant sur la spectroscopie d'absorption directe.

4. Procédé suivant l'une des revendications précédentes, reposant sur la spectroscopie à modulation en longueur d'onde, dans lequel on module le courant (i) de la diode (3) laser, en outre, à une amplitude petite et à au moins une fréquence supplémentaire, qui est choisie si basse qu'une modulation en longueur d'onde n'a pas lieu.

5. Procédé suivant la revendication 3 et 4, **caractérisé en ce que** l'on effectue des mesures, sur la base de la spectroscopie d'absorption directe et de la spectroscopie de modulation en longueur d'onde, en même temps dans chaque intervalle d'échantillonnage ou en alternance à des intervalles d'échantillonnage successifs et on combine leurs résultats par formation d'une valeur moyenne en le résultat de mesure.

6. Procédé suivant la revendication 4 ou 5, **caractérisé en ce que**, lors de la modulation à plusieurs fréquences supplémentaires, celles-ci sont à distance les unes des autres du double de la valeur de la fréquence supplémentaire la plus basse et **en ce que** l'exploitation, reposant sur la spectroscopie de modulation en longueur d'onde, du signal de mesure démodulé, a lieu aux points des deuxièmes harmoniques des fréquences supplémentaires et d'au moins l'une des fréquences somme et différence des fréquences supplémentaires.

7. Analyseur de gaz pour mesurer la concentration d'un constituant gazeux d'un gaz (1) à mesurer, comprenant
- une diode (3) laser accordable en longueur d'onde,
- une source (6) de courant alimentant la diode (3) laser en un courant (i),
- un générateur (7) de signal, qui commande la source (6) de courant pour faire varier le courant (i) pour l'échantillonnage en fonction de la longueur d'onde d'une raie d'absorption intéressante du constituant gazeux dans des intervalles d'échantillonnage de succédant périodiquement,
- un dispositif (9) de modulation, qui commande la source (6) de courant pour moduler sinusoïdalement le courant (i) à une fréquence et une amplitude données à l'avance,
- des moyens, qui font passer la lumière (4) modulée dans le gaz (1) à mesurer jusqu'à un détecteur (5) et
- un dispositif (15, 15') d'exploitation, qui démodule, à la fréquence de la modulation, un signal (14) de mesure produit par le détecteur (5) et exploite le signal (22) de mesure démodulé ainsi obtenu pour produire un résultat (16) de mesure,
**caractérisé**
- **en ce que** le dispositif (9) de modulation est constitué pour effectuer la module de la fréquence en fonction des propriétés de la diode laser à une fréquence si haute qu'une modulation en longueur d'onde n'a pas lieu et
- **en ce que** le dispositif (9) de modulation est constitué, en outre, pour effectuer la modulation de l'amplitude dans et en utilisant la plage de commande linéaire de la diode laser à un niveau maximum.

8. Analyseur de gaz suivant la revendication 7, **caractérisé**
- **en ce que** le dispositif (9) de modulation est constitué, en outre, pour moduler le courant (i) de la diode (3) laser à au moins une autre fréquence, qui est choisie également si haute qu'une modulation en longueur d'onde n'a pas lieu,
- **en ce que** les amplitudes de modulation sont choisies dans et en utilisant la plage de commande linéaire de la diode (3) laser à un niveau maximum,
- **en ce que** le dispositif (15, 15') d'exploitation est constitué pour démoduler le signal (14) de mesure produit par le détecteur (5) supplémentairement à la fréquence de la au moins une autre modulation et pour exploiter les signaux (22) de mesure démodulés obtenus, soit individuellement, et ensuite les réunir en le résultat (16) de mesure, soit les réunir d'abord et ensuite les exploiter en le résultat (16) de mesure.

9. Analyseur de gaz suivant la revendication 7 ou 8, **caractérisé en ce que** le dispositif (15, 15') d'exploitation est constitué pour exploiter le signal (22) de mesure démodulé sur la base de la spectroscopie d'absorption directe.

10. Analyseur de gaz suivant la revendication 7, 8 ou 9, **caractérisé**
- **en ce qu'**il y a un dispositif de modulation en basse fréquence (NF), qui commande la source (6) de courant pour moduler le courant (i) supplémentairement à une amplitude petite et à au moins une fréquence supplémentaire, qui est choisie si basse qu'une modulation en longueur d'onde n'a pas lieu et
- **en ce que** le dispositif (15, 15') d'exploitation est constitué pour exploiter le signal (14) de mesure démodulé sur la base de la spectroscopie de modulation en longueur d'onde.
